# EUROPEAN PATENT SPECIFICATION

(11) **EP 0 717 989 B1**
(45) Date of publication and mention of the grant of the patent: **10.01.2001**
(21) Application number: 94925490.8
(22) Date of filing: 30.09.1994
(51) Int. Cl.: A61K 9/51

(54) **PROCESS FOR COATING DROPLETS OR NANOMETRIC PARTICLES**
VERFAHREN ZUR UMHÜLLUNG VON NANOPARTIKELN ODER -TRÖPFCHEN
PROCEDE D'ENROBAGE DE GOUTTELETTES OU DE PARTICULES DE TAILLE NANOMETRIQUE

(30) Priority: 20.05.1994 ES 9401121
(43) Date of publication of application: 26.06.1996
(73) Proprietor: ALCON CUSI, S.A., El Masnou (Barcelona) (ES)
(72) Inventor: VALLET MAS, José Alberto, E-08022 Barcelona (ES); GALAN VALDIVIA, Francisco Javier, E-08915 Badalona (ES); CARRERAS PERDIGUER, Nuria, E-08140 Caldes de Montbui (ES)
(74) Representative: Ungria Lopez, Javier
(86) International application number: PCT/ES94/00085
(87) International publication number: WO 95/31975

(56) References cited:
- EP-A- 0 529 711

## Description

The present invention is comprised within the technical field of microencapsulation, particularly, in the coating of droplets or particles with sizes comprised within the nonometric range, using biodegradable and biocompatible polymers of different nature. The thus obtained products have important applications in pharmacy, medicine, cosmetics, veterinary, chemical industry, agriculture etc.

### STATE OF THE ART

A method for obtaining a fine suspension of particles formed by a biodegradable polymer, polycaprolactone, by means of precipitation produced by a change of solvent, has been described in "Mechanism of the biodegradation of polycaprolactone" (1983), Jarret, P. et al. Polum Prep. (Am. Chem. Soc. div. Polum. Chem.) Vol. 24 No. 1, page. 32-33.

EP Patent 0274961B1 (which corresponds to U.S. Patent No. 5049322) describes a method for obtaining vesicular type, spherical particles having a size less than 500 nm. The method comprises the preparation of a phase containing a polymer, an oil and a substance to be encapsulated in a solution or dispersion. The phase is added under agitation to another phase formed by a non solvent of the polymer and of the oil, producing the precipitation of the polymer, whereafter the solvents are removed by lyophilization. As one phase is incorporated over another, it is necessary to increase the size of the reactor which contains the mixture with regard to the final volume desired. This implies the necessity of a scaling to adapt the manufacturing conditions. There exists the difficulty in large volumes that, once the mixture is formed, the polymer solvent is in contact with the nanocapsules during a long period, this producing the risks of redisolution of the coating, and extraction of the active encapsulated substance into the external phase. On the other hand, the removal of solvents by means of lyophilization is a slow and expensive process, with the additional disadvantage that when inflammable solvents are involved, it is highly dangerous.

The present invention relates to a method of coating already formed droplets or particles. According to this method it is not necessary to agitate the mixture, which is produced by incorporating the two phases in a device in which the mixture flows continuously, to produce immediate evaporation of the solvents. The elaboration and facility conditions (reaction volume) are always the same, independently of the final volume to be obtained, so that scaling is not required for producing industrial quantities. The solvent remains in contact with the recently coated vesicules during a very short period, so that redissolution of the coating and the possible extraction of the active principle into the external phase is avoided, whatever the volume to be prepared is.

FR A2 515960 describes a process for preparing biodegradable polyalkylcyanoacrylate nanocapsules by polymerization of the corresponding monomer. Said nanocapsules contain a biologically active substance. The disadvantage of this method is that it requires a polymerization step, due to which it can be used only with specific polymers. Besides this important limitation, this process involves the difficulty of having to control the polymerization and the possible existence of residual monomers which may, in some cases, be toxic. The present invention has the advantages that it does not require a polymerization, that it is a more rapid process and being applicable to a great number of polymers of diverse nature.

EP 0480 729 A1 discloses a process for coating oil droplets, containing active principles for oral administration, with a polysaccharide with chelating capacity (sodium alginate) which hardens on the addition of multivalent cations, whereby microcapsules with sizes over 1µm are produced. Finally, lyophilization a product in powder form is obtained. This method is limited to the use of polysaccharides with chelating capacity. Likewise, as sonication is necessary, the process is not applicable in respect of active substances which are degraded by ultrasonic action. Additionally, the use of a multivalent cation solution renders the use of the product for any administration other than oral administration difficult. The present invention provides coated droplets with sizes appreciably below 1µm, does neither require hardening agents, nor sonication, and results in a product which may be administered orally, parenterally, through the nose, eyes, skin, lungs or by any other form of administration.

According to a process described in EP 0462003 A1 microcapsules, having sizes comprised between 25 and 100 µm with oil inside are obtained by drying by atomization an oil/water emulsion formed by an active ingredient and a gastroresistant polymer aqueous solution, and producing a fine powder, by using an atomizer at a temperature of 140°C. The use of high temperatures is a disadvantage since it limits the use of this method when the substance to be encapsulated is thermosensitive. This method is only usable for water soluble polymers and additionally differs from the object of the present invention in that the sizes of the capsules obtained are much greater.

A process described in EP 0556917 A1 allows obtaining biodegradable microcapsules containing an active substance by ultrasonic atomization of a solution or suspension over a non solvent, in such a way that the coagulated droplets are transferred to a second non solvent. This method, besides being complicated and requiring various solvents and a special atomizer by sonication, only allows the preparation of microcapsules with sizes over 10 µm.

Unlike all previously mentioned prior art, the present invention is a method which allows the preparation of large quantities of coated nanometric particles without changing the conditions or facilities, and which is consequently easy to industrialize. This method allows the rapid and continuous coating of temperature- or sonication-sensitive active substances, resulting in a final product which is usable in any field, specially in the pharmacy and veterinary field.

### DESCRIPTION OF THE INVENTION

The present invention refers to a new process for coating droplets or particles with sizes below one micrometer which contain, or are formed of, one or various chemical or biologically active substances. In consequence, the present invention allows the preparation of particles or droplets coated by one or various biodegradable and/or biocompatible polymers with diameters comprised between 100 and 1000 nm, preferably between 200 and 500 nm.

For carrying out the present invention first a fine dispersion of droplets or particles is prepared. In the case of droplets, the active substance is dissolved in a lipidic substance (generally an oil) or in a substance having a fusion point below the temperature of the dispersing means. The droplets may also be constituted by the active substance itself. In case of solid particles, these may be the active substance itself or have the active substance dispersed inside. They may also be part of a microorganism or integral microorganisms with sizes below one micrometer. The dispersing phase is constituted by a solvent and a non solvent of the polymer which forms the coating and, optionally, contains one or more surfactant or suspensor agents (PHASE 1). The relationship between the solvent and the non solvent in PHASE 1 must be the adequate one, so that the coat-forming polymer does not precipitate when mixed with the phase which contains the polymer. The phase which contains the coat-forming polymer (PHASE 2) is prepared by dissolving the coat-forming polymer in a solvent equal to the one used as part of PHASE 1, or any other which is miscible in a high relationship with the solvent of the polymer used in PHASE 1.

Once PHASE 1 and PHASE 2 have been separately prepared, they are lead through separate tubes to a mixing zone, where they are made to contact continuously, without agitation or ultrasonication, in a constant proportion which avoids instantaneous precipitation of the polymer and a constant volume of the mixture. During mixing, the polymer is not deposited on the droplets or particles, though the deposition process may initiate. Deposition takes place instantaneously when the mixture is pulverized in an evaporation system with temperature and vacuum conditions allowing the rapid evaporation of the polymer solvent. This provides for the immediate deposition of the polymer around the droplets or particles. Optionally, a portion of the non solvent, or the totality of the same, may be eliminated until a concentrated or dry product is obtained.

The conduction of the phases towards the mixture device zone, may be carried out by means of any pumping system, or with the help of pressure or vacuum.

It is a characteristic of the process of the present invention that, once PHASE 1 and PHASE 2 have been prepared, the formation of the mixture, the pulverization of the mixture and the deposition of the polymer are carried out in a totally continuous and simultaneous manner.

The ratio of the solvent to the non solvent of the coat-forming polymer in the initial dispersion must be such that when in contact with the phase which contains the polymer in the solution, immediate deposition of the polymer is not produced. Where a polymer which has a tendency to precipitate in the mixture of the phases is used, the small dimensions of the mixing zone result in the entry of the phases in the mixing zone and their exit in a pulverized form through the other end being so rapid, that the polymer has no time to precipitate. In this way, an uncontrolled precipitation is avoided which would produce the formation of aggregates is avoided, and it is ensured that the coating is produced at the moment of pulverization or nebulization.

The solvent and the non solvent of the polymer in the initial dispersion, is selected depending on the chemical and physico-chemical characteristics of the polymer, or the oil or lipidic substance, and of the active substance to be incorporated.

If the coat-forming polymer is not soluble in water, the non solvent may be a more or less complex aqueous solution, and the solvent may be any organic solvent which is miscible in a high proportion in water and capable of dissolving the polymer. The solvent for the polymer may be, for instance, an alcohol such as ethanol, methanol, isopropanol, a ketone of low molecular weight such as acetone or methyl ethyl ketone or other solvents such as acetonitrile or tetrahydrofuran. Normally, the solvent for the polymer has a dielectric constant over 15.

When the polymer is soluble in an organic solvent and water soluble depending on the pH or temperature, the aqueous solution of the initial dispersion must be adjusted to a pH and/or temperature at which said polymer be insoluble to ensure the deposition of the polymer when the evaporation of the solvent is produced during the pulverization.

The lipidic substance to be dispersed in the water may be a natural oil such as coconut oil, soya oil, olive oil, castor-oil, a mixture of capric acid tristearates and capric acid with glycerol, a mixture of saturated and unsaturated acid fats C₁₂-C₁₈ where the main constituyent is the linolenic acid (48%), a mixture of unsaturated polyglycosylated glycols constituted by glycerols and polyethyleneglycol esters, a mixture of saturated polyglycosylated C₈-C₁₀ glycerols, a palmitate-ester of glycerol formed by mono, di and triglycerols of natural C₁₆ and C₁₈ fat acids or a mixture thereof, a mineral oil or a phospholipid.

Generally, the concentration of the lipidic substance in the final product is comprised between 0.1 and 10% (w/v), preferably between 0.5 and 5% (w/v).

The surfactant or emulsifyer of PHASE 1 may be selected from amphoteric agents such as soya or egg lecithin, anionic agents such as sodium laurylsulfate, cationic agents such as benzalkonium chloride, or non ionic agents such as sorbitane mono oleate, sorbitane monoestearate, a polysorbate or a copolymer of polyoxyethylene-polyoxypropylene, or a mixture thereof.

The suspending agent may be a dextran, polyvinylic alcohol, a cellulose derivative or a natural rubber such as xanthane rubber. Any of these may be used in combination with any of the previously mentioned surfactants.

The concentration of the surfactant or suspending agent in the final formula is comprised between 0.01 and 10% w/v).

In PHASE 2, the polymer used may be selected from synthetic polymers such as glycolydes derived from propiolactone, butyrolactone and the epsilocaprolactone; semisynthetic polymers such as cellulose acetobutyrate, ethylcelulose, hydroxypropylmethylcelulose acetophtalate; the acrylic acid copolymers and acrylic polymers, lactic acid copolymers with the glycol acid or polycaprolactone. Other polymers which may be employed are cellulose acetophtalate, polyanhydrides, polyalphahydroxy-acids and natural polymers.

The concentration of the coat-forming polymer in the organic phase is comprised between 0.01 and 10% (w/v).

There are different forms of mixing the two phases. It may be performed through two parallel tubes, producing the union in a concentric or "Y" shaped zone in such a way that the two phases are joined simultaneously. The volumes of the phases may be equal or the volume of one phase may be larger than the other. The mixing zone has, at the end opposed to that at which the phases are incorporated, a suitable device, by which the mixture is made to exit the mixing zone in powder form towards an evaporation system in which the solvent of the polymer is totally removed; optionally also a portion or the whole of the non solvent is also removed under reduced pressure and at a temperature below 50°C. The degree of vacuum and the temperature must be adjusted depending on the solvent of the polymer used. In this way, rapid evaporation of the solvent and immediate deposition of the polymer around the droplets or particles is ensured, and the formation of aggregates or the appearance of uncoated particles or droplets is avoided.

The product thus obtained may be used in suspension or dry powder form, be extruded, compressed or granulated and be used alone or forming part of a more complex blend.

An analysis is subsequently carried out of the experimental results obtained in some specific tests performed according to the process of the present invention.

### 1. Nanoemulsion coating tests without drug

In order to study the suitability of the process for coating droplets which is the object of the present invention various formulations are prepared with the purpose of checking if the polymer is mainly deposited around the oil droplets instead of individually precipitating in the form of nanospheres, in which case the greater part of the oil droplets would remain uncoated. For this purpose, the three types of products which could be formed, were separately prepared: nanocapsules, naoemulsions and nanospheres.
a) A nanoemulsion of a mixture of caprylic acid and caprynic acid triesters with polyepsiloncaprolactone coated glycerol was prepared according to the process specified in the description of the present invention.
b) A nanoemulsion mixture of the caprylic acid and caprynic acid triester with glycerol was prepared in the same manner as in previous section (a), but without adding polymer in the organic solution (PHASE 2) of the description of the present invention.
c) For the preparation of nanospheres, the process detailed in the description of the present invention was followed, except that only a mixture of solvents and non solvents of the coat-forming polymer (polyepsiloncaprolactone), without oil, was used as PHASE 1.

Determinations were made of the particle size, the polydispersity and the Z potential of the products resulting from (a), (b) and (c) with a Zetasizer 3 (Malvern Instruments England).

As may be observed in Table 1, the values of the average size and the polydispersity of the uncoated oil droplets are greater than those of the coated oil droplets and these, in turn, are greater than the nanospheres.

The Z potential (parameter which gives an idea of the electric load on the surface of the droplets and particles), is -18mV for coated droplets, -8mV for the free oil droplets and -14mV for the nanospheres.

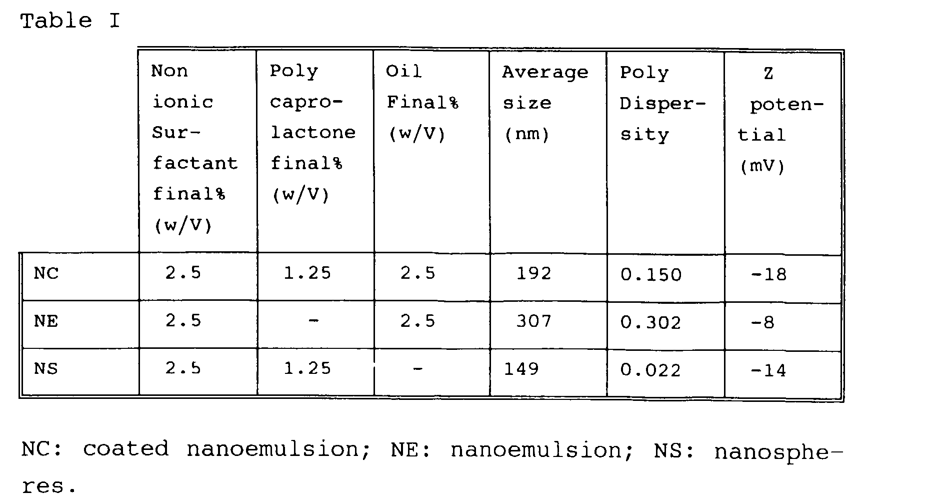

The values of size, polydispersity and Z potential correspond to the average of 10 measurements.

An evaluation was conducted by means of electronic microscopy at transmission of 66.000 magnification on diverse samples of the resultant products of (a) and (b) which were previously tinted with uranyl acetate at 1%.

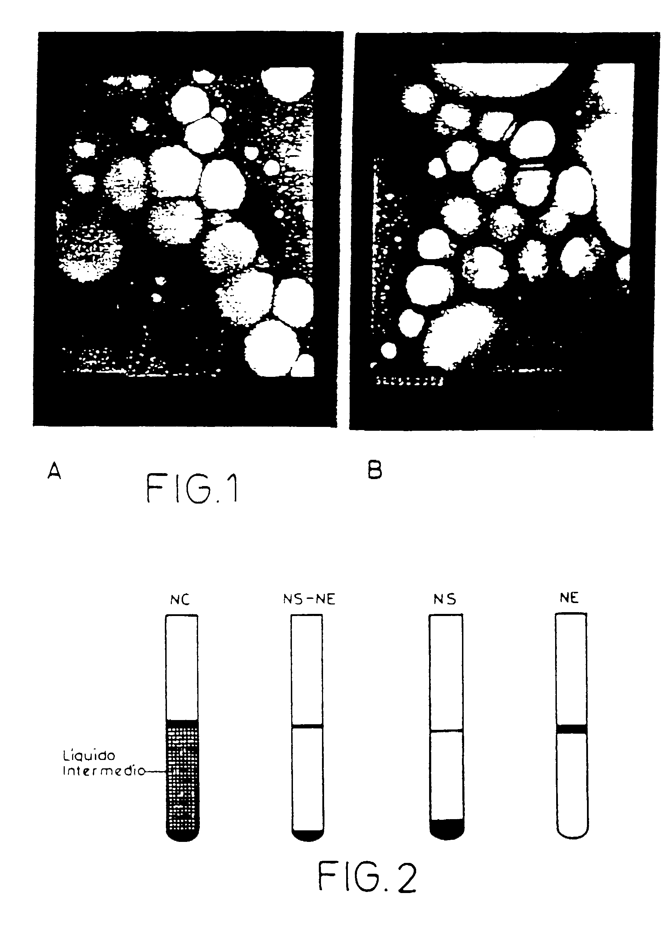

As can be observed in figure 1, the uncoated oil droplets (A), appear as uniform particles which adapt with one another, whilst coated oil droplets (B) appear as particles with a less dense core surrounded by a transparent zone limited by a dark edge (polymeric coating).

### 2. Nanoemulsion coating test with drug

The proceedings were similar to the previous section for the formulations without active principle, and a mixture of nanoemulsion and nanosphere was additionally prepared.
a) A nanoemulsion of a mixture of caprylic acid and caprinic acid triesters was prepared with polycaprolactone coated glycerol containing indomethacin at 0.1% (p/V) according the the process detailed in the description of the present invention.
b) A nanoemulsion of a mixture of caprylic acid and caprynic acid triesters was prepared with glycerol containing indomethacin at 0.1% (W/V) in the same manner as in previous section (a) but without adding polyepsiloncaprolactone in the organic solution PHASE 2 of the description of the present ivention.
c) For the preparation of indomethacin nanospheres at 0.1% (W/V), the process detailed in the description of the present invention was followed, except that only a mixture of solvent and non solvent of the coat-forming polymer (polyepsiloncaprolactone), without oil, was used as PHASE 1.

Additionally, a dispersion of oil droplets and nanoparticles was prepared, mixing at equal parts, the resultant products of previous sections (b) and (c)

Determinations were made of the particle size, the polydispersity and the Z potential with a Zetasizer 3 (Malvern Instruments, England), and 5 ml of each of the products was centrifuged during 2 cycles of lh at 4000 rpm in a Selecta Centromix centifuge.

The results are represented in Table II and in Figure 2. As may be observed in Table II, the average size values and the polydispersity values of the uncoated oil droplets are greater than those of the coated oil droplets and these, in turn, are greater than those of the nanospheres. The average size and the polydispersity of the nanosphere mixture and the uncoated oil droplets give intermediate values to those corresponding to the separate products and greater than those obtained for the coated droplets. Likewise, the nanosphere and nanoemulsion mixture showed a bimodal distribution (two populations of particle sizes). As regards the Z potential, the values obtained for the mixture of the nanospheres and the uncoated oil droplets, are comprised within the values corresponding to each product separately.

The Z potential of the coated droplets is greater (in absolute values) than those of the nanospheres, of the uncoated droplets and of their mixture. Consequently, the product obtained by the process which is the object of the present invention is not the result of a mixture of precipitated polymer particles (nanospheres) and of uncoated oil droplets.

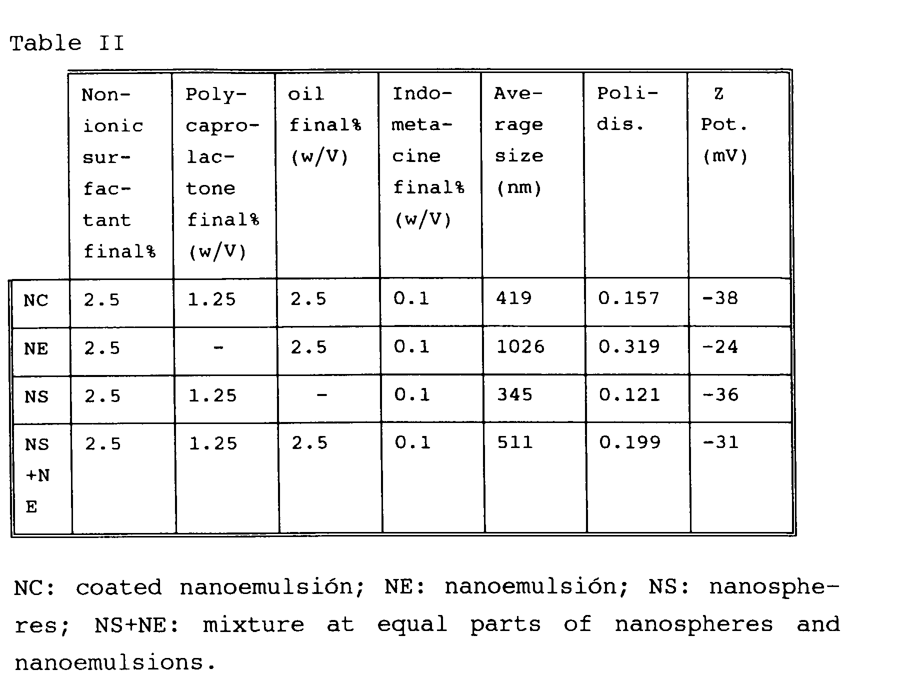

The values of size, polydispersity and Z potential correspond to the average of 10 measurements.

As may be observed in Figure 2, the nanospheres (NS) show a white sediment at the bottom of the tube, while the nanoemulsion (NE) shows a whitish float. On its part, the nanosphere and nanoemulsion mixture (NS+NE) presents at the same time, a sediment and a floating besides a practically transparent intermediate liquid. On the other hand, the coated oil droplets (NC) show a minimum sediment and floating but the intermediate liquid is much cloudier (whitish). This intermediate area, which is wider and cloudier, corresponds to the coated oil droplets with an intermediate density between that of the one corresponding to the oil droplets (less dense) and those of the nanospheres (denser).

### BRIEF DESCRIPTION OF THE FIGURES

Figure 1: (A) represents uncoated oil droplets which appear as uniform particles which adapt with one another; (B) represents the coated oil droplets which appear as particles with a denser core surrounded by a transparent zone limited by a dark edge (polymeric coat).

Figure 2 : Is a comparison of the appearance of the intermediate liquid in test 2, between the nanospheres (NS), the nanoemulsion (NE), the mixture of nanospheres and nanoemulsion (NS+NE) and the coated oil droplets (NC).

### EMBODIMENTS OF THE INVENTION

The present invention is additionally illustrated by means of the following examples, which must not be considered as limiting of the scope of the invention defined by the attached note of the claims:

For the description of the examples, commercial names of products which must be understood as being any product with the same characteristics, commercialized by any other Company were used. The products are as follows:
Miglyol 812® (Dynamit Nobel, Sweden): is a mixture of caprylic acid triesters and capyinic acid with glycerol.
Miglyol 840® (Dynamit Nobel, Sweden) is another mixture of caprylic triesters and caprynic ester with glycerol.
Edenor TiO₅® (Henkel, Dusseldorf): is a mixture of saturated and unsaturated fatty acids C₁₂-C₁₈ where the main constituent is the linolenic acid (48 %)
Eudragit® L 12 5 P Rohm Pharma, Darmstadt): is an anionic polymerizate of methacrylic acid and methyl methacrylate.
Lutrol F68 (BASF, Germany): is Poloxamer 188 which is a copolymer of polyoxyethylene and polyoxypropylene.

### EXAMPLE I

### NANOEMULSION OF MIGLYOL 812@ COATED WITH POLYEPSILON CAPROLACTONE

0.625 g of Lutrol F 68® was dissolved under agitation in 62 ml of deionized water and filtered through 0.22 µm. 0.625 g of Miglyol 812® was dissolved in 62 ml of acetone. The acetonic solution was incorporated to the initial aqueous solution under magnetic agitation so that a dispersion of droplets with an average size below 1µm was obtained (Phase 1), 0.312 g of polyepsiloncaprolactone was dissolved in 125 ml of acetone with the aid of ultrasonication (Phase 2). The two phases were continuously mixed by the two parallel tubes, maintaining the relation of the phases constant in the mixing zone and pulverizing the resultant mixture towards the evaporation system simultaneously to the formation of the mixture. In the evaporation system the acetone (polymer solvent) was removed, under reduced pressure and at a maximum temperature of 45^{º}C so that the deposition of the polymer around the oil droplets was produced and part of the water (non-solvent of the polymer) was removed, until a final volume of 25 ml is reached. The average size of the coated droplets, measured in a Zetasizer 3 (Malvern Instruments) was 192 ±0.1 nm.

### EXAMPLE 2

### NANOEMULSION OF MIGLYOL 812® COATED WITH POLYEP SILONCA-PROLACTONE

The technique described in Example 1 was followed, except that the ratio of solvents in the initial dispersion was of 2:3 water/acetone expressed in volumes, instead of 1:1 water/acetone. The average size of the coated droplets, measured in a Zetasizer 3 (Malvern Instruments), was of 307 ± 0.5 nm.

### EXAMPLE 3

### NANOEMULSION OF MIGLYOL 812® COATED WITH POLYLACTIC-GLYCOLIC COPOLIMER 75:25

The technique described in example 1 was followed, except that 0.830g of Lutrol F68®, 0.207g of polylactic-glycolic copolymer were used instead of polyepsiloncaprolactone and 0.415g of Miglyol 812®. The average size of the coated droplets, measured in a Zetasizer 3 (Malvern Instruments) was of 197 ±5nm.

### EXAMPLE 4

### NANOEMULSION OF CARTEOLOL BASE AT 0.2% COATED WITH POLYEPSILONCAPROLACTONE

0.375g of Lutrol F68® was dissolved in 40ml of deionized water and filtered through 0.22µm under agitation. 0.030g of carteolol base was dissolved in 0.375g of Edenor Ti0₅® and the resultant solution was added to 60ml of acetone. The acetonic solution was incorporated into the initial aqueous solution under magnetic agitation to obtain a dispersion of droplets with an average size below 1µm (Phase 1). 0.187g of polyepsiloncaprolactone was dissolved in 100ml of acetone with the aid of ultrasonication. (Phase 2). The two phases were continuously mixed through the two parallel tubes, maintaining the ratio of the phases constant in the mixing zone, and pulverizing the resultant mixture towards the evaporation system simultaneously with the formation of the mixture. In the evaporation system, the acetone (solvent of the polymer) was removed, under reduced pressure and at a maximum temperature of 45^{º}C so that the deposition of the polymer around the oil droplets was produced, and part of the water (non solvent of the polymer) was removed until a final volume of 25ml is reached. The average size of the coated droplets, measured in a Zetasizer 3 (Malvern Instruments), was 375 ±3nm.

For separating the coated droplets from the external aqueous phase, the ultrafiltering-centrifugal technique was used, determining, by means of HPLC, the concentration of carteolol in the total formula and in the filtrate. The percentage of encapsulation of the carteolol was calculated by the difference between the concentration in the total formula and that of the filtrate. The percentage of encapsulation was 70%.

### EXAMPLE 5

### NANOEMULSION OF INDOMETHACIN AT 0.1% COATED WITH POLYEPSILONCAPROLACTONE.

1.66g of Lutrol F68® was dissolved in 100ml of deionized water and filtered through 0.22µm under agitation. 0.050g of indomethacin was dissolved in 0.830g of Miglyol 812® applying heat, and the resulting solution is added to 100ml of acetone. The acetonic solution was incorporated into the initial aqueous solution under magnetic agitation so as to obtain a dispersion of droplets with an average size below 1µm (Phase 1). 0.415g of polyepsiloncaprolactone was dissolved in 200ml of acetone with the aid of ultrasonication (Phase 2). The two phases are mixed continuously through the two parallel tubes, maintaining the ratio of the phases constant in the mixing zone and pulverizing the resultant mixture towards the evaporation system simultaneously with the formation of the mixture. > In the evaporation system the acetone (solvent of the polymer) was removed under reduced pressure and at a maximum temperature of 45^{º}C, so that deposition of the polymer around the oil droplets was produced, and part of the water (non solvent of the polymer) was removed until a final volume of 50ml is reached. The final pH is adjusted to 5.5 with H Cl 0.1 M. The average size of the coated droplets, measured in a Zetasizer 3 (Malvern Instruments) was, 551 ±15nm.

For the separation of the coated droplets from the external aqueous phase, ultrafiltering-centrigu- gal technique was used, determining by means of HPLC, the concentration of indomethacin in the total formula and in the filtrate. The percentage of encapsulation of the indomethacin was calculated by the difference between the concentration in the total formula and that of the filtrate. The percentage of encapsulation was 99%.

### EXAMPLE 6

### NANOEMULSION OF MIGLYOL 840® COATED WITH EUDRAGIT L 12.5 P®

0.375G of Lutrol F68⁸ was dissolved under agitation in 40ml of deionized water and filtered through 0.22µm. The pH was adjusted to 4.5 with HCl 0.1M. 0.375g of Miglyol 840® was dissolved in 60 ml of acetone. The acetonic solution was incorporated into the initial aqueous solution under magnetic agitation so that a dispersion of droplets with an average size below 1 µm was obtained (Phase 1). 0.150 g of Eudragit L 12.5 P® was dissolved in 100 ml of acetone (Phase 2). The two phases were continuously mixed through the two parallel tubes, maintaining constant the ratio of phases in the mixing zone and pulverizing the resultant mixture towards the evaporation system simultaneously with the formation of the mixture. In the evaporation system, the acetone (solvent of the polymer) was removed under reduced pressure and at a maximum temperature of 45^{º}C so that the deposition of the polymer around the oil droplets was produced, and part of the water (non solvent of the polymer) was removed, until a final volume of 15ml was reached. The average size of the coated droplets, measured in a Zetasizer 3 (Malvern Instruments) was, 832 ± nm.

### EXAMPLE 7

### NANOEMULSION OF CARTEOLOL AT 0.1% COATED WITH EUDRAGIT L 12.5 P®

The technique described in example 6 was followed, except that Miglyol 840® was replaced by Edenor Ti0ₛ®, and 0.030 g of carteolol base was included in the oil. The average size of the coated droplets measured in a Zetasizer 3 (Malvern Instruments), was 290 ± 12nm.

For the separation of the coated droplets of the external aqueous phase ultrafiltering-centrifugal technique was used, determining, by means of HPLC, the carteolol concentration in the total formula and in the filtrate. The percentage of encapsulation of the carteolol was calculated by the difference between the concentration in the total formula and that of the filtrate. The percentage of encapsulation was of 66%.

### EXAMPLE 8

### POLYSTYRENE LATEX COATED WITH POLYEPSILONCAPROLACTONE

0.125g of Lutrol F68® was dissolved under agitation in 40 ml of deionized water and filtered through 0.22µm. To the previous solution 100 µm of polystyrene latex with an average particle size of 200nm and a Z potential of -30.81 mV measured in a Zetasizer 3 (Malvern Instruments) was added, and subsequently 20 ml of acetone were added, to obtain a dispersion of droplets with average size below 1µm (Phase 1). 0.01g of polyepsiloncaprolactone was dissolved, by means of ultrasonication, in 25ml of acetone (Phase 2) The two phases were continuously mixed through the two parallel tubes, maintaining the relationship of the phases constant in the mixing zone and pulverizing the resultant mixture towards the evaporation system simultaneously with the formation of the mixture. In the evaporation system the acetone (solvent of the polymer) was removed under reduced pressure and at a maximum temperature of 45°C, in order to produce the deposition of the polymer around the latex particles and part of the water (not the solvent of the polymer) was removed until a final volume of 7ml was reached. The average size of the coated particles, measured in a Zetasizer 3 (Malvern Instruments), was 286 ± 9nm and the Z potential was 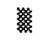 14.13 mV ± 1.5 mV.

### EXAMPLE 9

### POYSTYRENE LATEX COATED WITH EUDRAGIT L 12.5 P

The same procedure as for example 8 was followed, except that polyepsiloncaprolactone was replaced by Eudragit L 12.5 P. The initial solution of water and Lutrol F68 was adjusted to approximately pH 4. The average size of the coated particles, measured in a Zetasizer 3 (Malvern Instruments) was of 270 ± 12nm and the Z potential of - 17.39 ± 1.5 mV.

## Claims

1. A process for providing a coating to nanospheres having sizes below 1 micron, selected from droplets or solid particles containing or being formed of a chemical or a biologically active substance, with a coating formed by at least one biocompatible polymer, the process being constituted by the steps of
preparing a first phase constituted of a fine dispersion of nanospheres in a solution of a first solvent and a non solvent for the polymer forming the coating, the first phase further containing a surfactant or a suspending agent;
preparing a second phase containing at least one polymer dissolved in a second solvent or in a mixture of miscible solvents;
mixing the first and second phases continuously in a mixing zone while maintaining a constant ratio of said phases and a constant volume of said phases in the mixing zone; and
evaporating at least a portion of the solvents;
whereby
at least one operating condition selected from the ratio of solvent and non solvent for the polymer, pH and temperature in the first phase, is selected such that when the first phase is mixed with the second phase, no immediate deposition of the polymer is produced;
mixing of the first and second phase is carried out without agitation and without ultrasonication; and
evaporation is carried out by spraying the resulting mixture into an evaporation system where it is converted into a pulverized form by removal of at least a portion of the solvents under reduced pressure so that the polymer is deposited around the nanospheres.

2. A process according to claim 1, characterized in that, after solvent evaporation, part or all of the non solvents for the polymer, is removed to obtain a concentrated or dry final product.

3. A process according to any one of claims 1 or 2, characterized in that the polymer forming the coating is biodegradable and/or biocompatible.

4. A process according to claim 1, characterized in that the active substance is a drug, a forerunner of a drug or a cosmetic substance.

5. A process according to any one of claims 1 or 4, characterized in that the active substance is a biologically active product or a microorganism or fragments of microorganisms.

6. A process according to any one of claims 1, 4 or 5, characterized in that the active substance is dissolved or dispersed in the droplets or particles.

7. A process according to any one claims 1 or 4-6, characterized in that the active substance forms the nanospheres.

8. A process according to any one of claims 1 or 7, characterized in that the nanospheres are droplets formed by an oil or by a lipidic substance

9. A process according to claim 1, characterized in that the mixing, the spraying and the deposition of the polymer is carried out continuously and simultaneously in time.

10. A process according to claim 1, characterized in that the concentration of the surfactant agent is comprised between 0.01% and 10% (w/V).

11. A process according to claim 1, characterized in that the concentration of the suspending agent is comprised between 0.1% and 10% (w/V).

12. A process according to claim 1, characterized in that the solvent for the polymer forming the coating is miscible in the non solvent in any proportion.

13. A process according to any of claims 1 or 12, characterized in that the solvent for the polymer forming the coating has a dielectric constant above 15.

14. A process according to any one of claims 1, 3, 12 or 13, characterized in that the concentration of the polymer forming the coating is 0.01% to 10% (w/V).

15. A process according to claim 2, characterized in that the final product is lyophilized, extruded or compressed.

## Patentansprüche

1. Verfahren zum Bereitstellen eines Überzugs auf Nanokugeln (nanospheres) mit Größen unter 1 µm, ausgewählt aus Tröpfchen oder festen Teilchen, die eine Chemikalie oder eine biologisch wirksame Substanz enthalten oder daraus gebildet sind, mit einem Überzug, der durch mindestens ein biokompatibles Polymer gebildet ist, wobei das Verfahren die folgenden Schritte beinhaltet:
Herstellen einer ersten Phase, die eine feine Dispersion von Nanokugeln in einer Lösung von einem ersten Lösungsmittel und einem Nichtlösungsmittel für das Polymer, welches den Überzug bildet, beinhaltet, wobei die erste Phase außerdem einen oberflächenaktiven Stoff oder ein Suspendiermittel enthält;
Herstellen einer zweiten Phase, die mindestens ein Polymer in einem zweiten Lösungsmittel oder in einem Gemisch aus mischbaren Lösungsmitteln gelöst enthält;
Vermischen der ersten und zweiten Phase kontinuierlich in einer Mischzone, während ein konstantes Verhältnis der Phasen und ein konstantes Volumen der Phasen in der Mischzone aufrechterhalten wird; und
Verdampfen zumindest eines Teils der Lösungsmittel;
wobei
mindestens eine Betriebsbedingung, ausgewählt aus dem Verhältnis von Lösungsmittel und Nichtlösungsmittel für das Polymer, dem pH und der Temperatur in der ersten Phase, so gewählt wird, dass, wenn die erste Phase mit der zweiten Phase vermischt wird, keine unmittelbare Abscheidung des Polymers erfolgt;
das Vermischen der ersten und zweiten Phase ohne Rühren und ohne Ultrabeschallung erfolgt; und
das Verdampfen durch Versprühen des resultierenden Gemisches in ein Verdampfungssystem erfolgt, wo es durch Entfernen von zumindest einem Teil der Lösungsmittel unter vermindertem Druck in eine pulverisierte Form umgewandelt wird, so dass das Polymer um die Nanokugeln herum abgeschieden wird.

2. Verfahren nach Anspruch 1, **dadurch gekennzeichnet,** dass nach der Lösungsmittelverdampfung ein Teil der oder die gesamten Nichtlösungsmittel für das Polymer entfernt werden, um ein konzentriertes oder trockenes Endprodukt zu erhalten.

3. Verfahren nach einem der Ansprüche 1 oder 2, **dadurch gekennzeichnet,** dass das Polymer, das den Überzug bildet, biologisch abbaubar und/oder biokompatibel ist.

4. Verfahren nach Anspruch 1, **dadurch gekennzeichnet,** dass die wirksame Substanz ein Arzneimittel, eine Vorläufersubstanz eines Arzneimittels oder eine kosmetische Substanz ist.

5. Verfahren nach einem der Ansprüche 1 oder 4, **dadurch gekennzeichnet,** dass die wirksame Substanz ein biologisch wirksames Produkt oder ein Mikroorganismus oder Fragmente von Mikroorganismen ist.

6. Verfahren nach einem der Ansprüche 1, 4 oder 5, **dadurch gekennzeichnet,** dass die wirksame Substanz in den Tröpfchen oder Teilchen aufgelöst oder dispergiert ist.

7. Verfahren nach einem der Ansprüche 1 oder 4-6, **dadurch gekennzeichnet,** dass die wirksame Substanz die Nanokugeln bildet.

8. Verfahren nach einem der Ansprüche 1 oder 7, **dadurch gekennzeichnet,** dass die Nanokugeln Tröpfchen sind, die durch ein Öl oder durch eine Lipidsubstanz gebildet werden.

9. Verfahren nach Anspruch 1, **dadurch gekennzeichnet,** dass das Vermischen, das Versprühen und die Abscheidung des Polymers kontinuierlich und gleichzeitig erfolgt.

10. Verfahren nach Anspruch 1, **dadurch gekennzeichnet,** dass die Konzentration des oberflächenaktiven Stoffes zwischen 0,01 % und 10 % (Gew.Vol.) liegt.

11. Verfahren nach Anspruch 1, **dadurch gekennzeichnet,** dass die Konzentration des Suspendiermittels zwischen 0,1 % und 10 % (Gew.Vol.) liegt.

12. Verfahren nach Anspruch 1, **dadurch gekennzeichnet,** dass das Lösungsmittel für das Polymer, welches des Überzug bildet, in dem Nichtlösungsmittel in jedem Verhältnis mischbar ist.

13. Verfahren nach einem der Ansprüche 1 oder 12, **dadurch gekennzeichnet,** dass das Lösungsmittel für das Polymer, welches den Überzug bildet, eine Dielektrizitätskonstante über 15 aufweist.

14. Verfahren nach einem der Ansprüche 1, 3, 12 oder 13, **dadurch gekennzeichnet,** dass die Konzentration des Polymers, welches den Überzug bildet, 0,01 % bis 10 % (Gew.Vol.) beträgt.

15. Verfahren nach Anspruch 2, **dadurch gekennzeichnet,** dass das Endprodukt lyophilisiert, extrudiert oder komprimiert wird.

## Revendications

1. Procédé d'enrobage de nanosphères ayant des tailles inférieures à 1 micron, choisies parmi des gouttelettes ou des particules solides contenant, ou formées de, un produit chimique ou une substance biologiquement active, avec un enrobage formé par au moins un polymère biocompatible, le procédé comprenant les étapes consistant à :
préparer une première phase constituée d'une fine dispersion de nanosphères dans une solution d'un premier solvant et d'un non-solvant pour le polymère formant l'enrobage, la première phase contenant en plus un agent tensio-actif ou un agent de suspension ;
préparer une deuxième phase contenant au moins un polymère dissous dans un deuxième solvant ou dans un mélange de solvants miscibles ;
mélanger les première et deuxième phases en continu dans une zone de mélangeage tout en maintenant un ratio constant desdites phases et un volume constant desdites phases dans la zone de mélangeage ; et
évaporer au moins une partie des solvants ;
dans lequel
au moins une condition opératoire, choisie parmi le ratio de solvant et de non-solvant pour le polymère, le pH et la température dans la première phase, est choisie telle que, lorsque la première phase est mélangée avec la deuxième phase, il ne se produit pas de dépôt immédiat du polymère ;
le mélangeage de la première et de la deuxième phase se fait sans agitation et sans traitement aux ultrasons ; et
l'évaporation se fait en pulvérisant le mélange résultant dans un système d'évaporation où il est converti en une forme pulvérisée par enlèvement d'au moins une partie des solvants sous pression réduite de telle sorte que le polymère se dépose autour des nanosphères.

2. Procédé selon la revendication 1, caractérisé en ce que, après l'évaporation des solvants, la totalité ou une partie des non-solvants pour le polymère est enlevée afin d'obtenir un produit final concentré ou sec.

3. Procédé selon l'une quelconque des revendications 1 ou 2, caractérisé en ce que le polymère formant l'enrobage est biodégradable et/ou biocompatible.

4. Procédé selon la revendication 1, caractérisé en ce que la substance active est un médicament, un précurseur d'un médicament ou une substance cosmétique.

5. Procédé selon l'une quelconque des revendications 1 ou 4, caractérisé en ce que la substance active est un produit biologiquement actif ou un micro-organisme ou des fragments de micro-organismes.

6. Procédé selon l'une quelconque des revendications 1, 4 ou 5, caractérisé en ce que la substance active est dissoute ou dispersée dans les gouttelettes ou les particules.

7. Procédé selon l'une quelconque des revendications 1 ou 4 à 6, caractérisé en ce que la substance active forme les nanosphères.

8. Procédé selon l'une quelconque des revendications 1 ou 7, caractérisé en ce que les nanosphères sont des gouttelettes formées par une huile ou par une substance lipidique.

9. Procédé selon la revendication 1, caractérisé en ce que le mélangeage, la pulvérisation et le dépôt du polymère se font en continu et de manière simultanée dans le temps.

10. Procédé selon la revendication 1, caractérisé en ce que la concentration de l'agent tensio-actif est comprise entre 0,01% et 10% (m/V).

11. Procédé selon la revendication 1, caractérisé en ce que la concentration de l'agent de suspension est comprise entre 0,1% et 10% (m/V).

12. Procédé selon la revendication 1, caractérisé en ce que le solvant pour le polymère formant l'enrobage est miscible avec le non-solvant en n'importe quelle proportion.

13. Procédé selon l'une quelconque des revendications 1 ou 12, caractérisé en ce que le solvant pour le polymère formant l'enrobage a une constante diélectrique supérieure à 15.

14. Procédé selon l'une quelconque des revendications 1, 3, 12 ou 13, caractérisé en ce que la concentration du polymère formant l'enrobage est de 0,01% à 10% (m/V).

15. Procédé selon la revendication 2, caractérisé en ce que le produit final est lyophilisé, extrudé ou comprimé.
